# EUROPEAN PATENT APPLICATION

(11) **EP 3 656 762 A1**
(43) Date of publication of application: **27.05.2020**
(21) Application number: 18382856.5
(22) Date of filing: 26.11.2018
(51) Int. Cl.: C07D 209/52, A61K 31/403, A61P 29/00

(54) **COMPOUNDS FOR THE TREATMENT OF NF-KB-RELATED DISEASES OR DISORDERS**

(71) Applicant: Instituto Biomar S.A., 24009 Armunia, León (ES); Universidad Autónoma de Madrid, 28049 Madrid (ES)
(72) Inventor: FERNÁNDEZ MEDARDE, Antonio, 24009 Armunia, León (ES); VINUESA NAVARRO, María de los Ángeles, 24009 Armunia, León (ES); SÁNCHEZ LÓPEZ, José María, 24009 Armunia, León (ES); CAÑEDO HERNÁNDEZ, Librada María, 28770 Colmenar Viejo, Madrid (ES); FRESNO ESCUDERO, Manuel, 28049 Madrid (ES); SÁNCHEZ -VALDEPEÑAS VILLEGAS, Carmen María, 28049 Madrid (ES)
(74) Representative: ABG Intellectual Property Law, S.L.

(57) **Abstract**

The invention relates to a family of fungal compounds which are inhibitors of NF-κB inducing kinase and are thus useful in the treatment of NF-κB-related diseases or disorders such as inflammatory or autoimmune diseases or disorders.

## Description

### FIELD OF THE INVENTION

The present invention relates to the field of medicine, and more particularly to the treatment of NF-κB-related diseases or disorders such as inflammatory or autoimmune diseases or disorders.

### BACKGROUND OF THE INVENTION

NF-κB is a heterodimeric transcription factor which is present in the cytosol of cells. When activated, NF-κB migrates into the nucleus of the cell and controls the expression of multiple genes involved in inflammatory and immune responses. Examples of such genes are those encoding proinflammatory cytokines TNF-α, IL-1β, IL-6 or IL-8.

A number of inflammatory and immune diseases and disorders have been associated to the dysregulation, and in particular to the overactivation, of NF-κB. Important examples are rheumatoid arthritis, multiple sclerosis or MS, asthma, inflammatory bowel disease or IBL (Tak et al., J Clin Invest, 2001, 107(1): 7-11; Liu et al., Signal Transduction and Targeted Therapy, 2017, 2, e17023, 1-9; Agosti et al., Lung Biologu in Health and Disease, 167, 2002, p363-365), transplantation rejection (Molinero et al. Transplant Rev (Orlando), 2012, 26(3):189-200), psoriasis (Goldminz et al., J Dermatol Sci, 2013, 69(2):89-94), dermatitis (Tanaka et al., J Invest Dermatol, 2007, 127(4):855-63), systemic lupus erythematosus or SLE (Brightbill et al., Nature Communications, 2018, 9:179) or type 2 diabetes (Sheng. et al, Nat Med, 2012, 18(6): 943-949).

The NF-κB pathway has therefore become an interesting therapeutic target for controlling the development of such diseases. A number of compounds with immunosuppressive and anti-inflammatory properties have been studied as inhibitors of said pathway, e.g. glucocorticoids, cyclosporin A, tacrolimus, rapamycin or salicylates.

Specific attention has been paid in the art to the dampening of NF-κB activity *via* inhibition of NF-κB inducing kinase (NIK, *aka* MAP3K14). NIK is a serine/threonine kinase which plays an essential role in regulating NF-κB activation and therefore by itself constitutes a key therapeutic target in the NF-κB pathway. Document WO 2016/062792 refers to the involvement of NIK in inflammation and allo or autoinmmunity, with particular reference to rheumatoid arthritis, IBL, sepsis, colonic inflammation, chronic obstructive pulmonary disease, diabetes-associated inflammation, obesity-associated inflammation, insulin resistance, transplantation rejection and GVHD. Further examples of diseases associated specifically to NIK activity are MS (Lacher et al. Journal of Neuroimmunology, 2014, 275(1):202), SLE (Brightbill et al., Nature Communications, 2018, 9(1):179), liver injury and liver fibrosis (Shen et al., Hepatology, 2014, 60(6):2065-2076), or psoriasis (Huang et al., The Journal of Experimental Medicine, 2018, 215(8)).

A constant need exists to enlarge the plethora of molecules that can be employed to regulate NF-κB and thus serve as active agents in the treatment of inflammatory and immune diseases.

### SUMMARY OF THE INVENTION

It has now surprisingly been found that a series of fungal compounds and their derivatives are capable of inhibiting NF-κB inducing kinase (NIK), and are hence useful in the treatment of NF-κB-related diseases or disorders such as inflammatory or autoimmune diseases or disorders.

Thus, the present invention refers in a first aspect to a compound of formula (I) 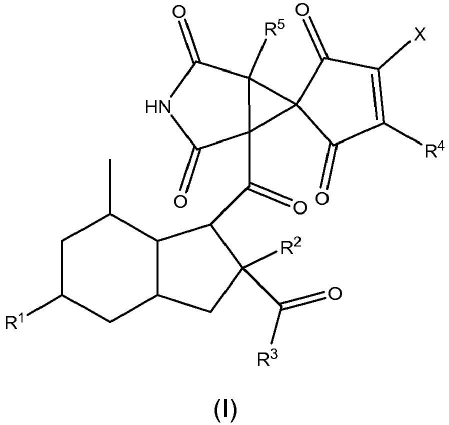 wherein
R¹ is selected from -C(=O)OR^{a}, -C(=O)NHR^{a}, -C(=O)N(R^{a})₂, -C(=O)R^{a}, and-CH₂OR^{a},
   wherein each R^{a} is independently selected from hydrogen, and substituted or unsubstituted alkyl, alkenyl or alkynyl group;
R² is selected from R^{c}, -OR^{c}, -NHR^{c} and -N(R^{c})₂,
   wherein each R^{c} is independently selected from hydrogen, and substituted or unsubstituted alkyl, alkenyl or alkynyl group;
R³, R⁴ and R⁵ are each independently selected from substituted or unsubstituted alkyl, alkenyl and alkynyl group;
X is H or halogen;
or a pharmaceutically acceptable salt, stereoisomer, or solvate thereof;
for use in the treatment of a NF-κB-related disease or disorder, with the proviso that the disease or disorder is not cancer.

The present invention similarly relates to a pharmaceutical composition comprising a compound of formula (I) or a pharmaceutically acceptable salt, stereoisomer, or solvate thereof according to the present invention, and a pharmaceutically acceptable excipient; for use in the treatment of a NF-κB-related disease or disorder, with the proviso that the disease or disorder is not cancer.

The present invention also refers to a method for treating a NF-κB-related disease or disorder in a subject in need thereof, the method comprising administering to the subject a compound of formula (I) or a pharmaceutically acceptable salt, stereoisomer, or solvate thereof according to the present invention, or a pharmaceutical composition according to the present invention, with the proviso that the disease or disorder is not cancer.

The present invention also refers to the use of a compound of formula (I) or a pharmaceutically acceptable salt, stereoisomer, or solvate thereof according to the present invention, or of a pharmaceutical composition according to the present invention, in the manufacture of a medicament for the treatment of a NF-κB-related disease or disorder, with the proviso that the disease or disorder is not cancer.

The present invention also refers to the use of a compound of formula (I) or a pharmaceutically acceptable salt, stereoisomer, or solvate thereof according to the present invention, or of a pharmaceutical composition according to the present invention, in the treatment of a NF-κB-related disease or disorder, with the proviso that the disease or disorder is not cancer.

These aspects as well as preferred embodiments thereof are further described hereinafter in the detailed description and in the claims.

### BRIEF DESCRIPTION OF THE DRAWINGS

**Figure 1****.** Luciferase activity in Jurkat cells transiently transfected with a luciferase reporter driven by CD28RE, along with equivalent amounts of NIK and c-Rel expression constructs and then treated with 0 (C), 0.5, 1 and 2.5 µg/ml of Compound 1.
**Figure 2****.** EAE development in mice following MOG injection in animals treated with Vehicle or Compound 1 (Test item A; 3 mg/kg), or with Vehicle or Cyclosporine (10 mg/kg), both i.p. daily from day 0.

### DETAILED DESCRIPTION OF THE INVENTION

As set out in the brief summary of the invention, the present invention involves the use of a compound of formula (I) 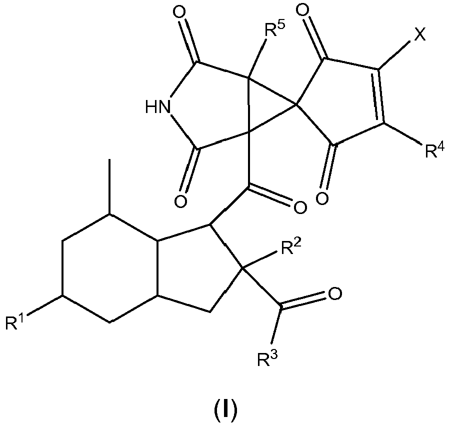 wherein
R¹ is selected from -C(=O)OR^{a}, -C(=O)NHR^{a}, -C(=O)N(R^{a})₂, -C(=O)R^{a}, and-CH₂OR^{a},
   wherein each R^{a} is independently selected from hydrogen, and substituted or unsubstituted alkyl, alkenyl or alkynyl group;
R² is selected from R^{c}, -OR^{c}, -NHR^{c} and -N(R^{c})₂,
   wherein each R^{c} is independently selected from hydrogen, and substituted or unsubstituted alkyl, alkenyl or alkynyl group;
R³, R⁴ and R⁵ are each independently selected from substituted or unsubstituted alkyl, alkenyl and alkynyl group;
X is H or halogen;
or a pharmaceutically acceptable salt, stereoisomer, or solvate thereof.

In the context of the present invention, the following terms have the meaning detailed below.

"Alkyl" refers to a straight or branched hydrocarbon chain radical containing no unsaturation (double or triple bond), and which is attached to the rest of the molecule by a single bond. Typical alkyl groups have from 1 to about 12, 1 to about 8, or 1 to about 6 carbon atoms, e. g., methyl, ethyl, n-propyl, i-propyl, n-butyl, t-butyl, n-pentyl, etc.

"Alkenyl" refers to a straight or branched hydrocarbon chain radical containing at least two carbon atoms and at least one unsaturation, and which is attached to the rest of the molecule by a single bond. Typical alkenyl radicals have from 2 to about 12, 2 to about 8 or 2 to about 6 carbon atoms. In a particular embodiment, the alkenyl group is vinyl, 1-methyl-ethenyl, 1-propenyl, 2-propenyl, or butenyl.

"Alkynyl" refers to a straight or branched hydrocarbon chain radical containing at least two carbon atoms and at least one carbon-carbon triple bond, and which is attached to the rest of the molecule by a single bond. Typical alkynyl radicals have from 2 to about 12, 2 to about 8 or 2 to about 6 carbon atoms. In a particular embodiment, the alkynyl group is ethynyl, propynyl (e.g. 1-propynyl, 2-propynyl), or butynyl (e.g. 1-butynyl, 2-butynyl, 3-butynyl).

"Halogen", "halo" or "hal" refers to bromo, chloro, iodo or fluoro.

The above mentioned groups that may be substituted are substituted at one or more available positions by one or more groups selected from R^{e}, -OR^{e}, =O, -SR^{e}, -SOR^{e}, -SO₂R^{e}, -OSO₂R^{e}, -OSO₃R^{e}, -NO₂, -NHR^{e}, -N(R^{e})₂, =N-R^{e},-N(R^{e})COR^{e}, -N(COR^{e})₂, -N(R^{e})SO₂R^{e}, -N(R^{e})C(=NR^{e})N(R^{e})R^{e}, -N₃, -CN, halogen, -COR^{e}, -COOR^{e}, -OCOR^{e}, -OCOOR^{e}, -OCONHR^{e}, -OCON(R^{e})₂,-CONHR^{e}, -CON(R^{e})₂, -CON(R^{e})OR^{e}, -CON(R^{e})SO₂R^{e}, -PO(OR^{e})₂, -PO(OR^{e})R^{e}, -PO(OR^{e})(N(R^{e})R^{e}), aryl and heterocyclyl, wherein each R^{e} is independently selected from hydrogen, and substituted or unsubstituted alkyl, alkenyl or alkynyl group. "Aryl" refers to single and multiple ring radicals, including multiple ring radicals that contain separate and/or fused aryl groups. Typical aryl groups contain from 1 to 3 separated and/or fused rings and from 6 to about 18 carbon ring atoms, preferably from 6 to about 14 carbon ring atoms, such as phenyl, 1- or 2-naphthyl, biphenyl, indenyl, fenanthryl or anthracyl radical. "Heterocyclyl" include heteroaromatic and heteroalicyclic groups containing from 1 to 3 separated and/or fused rings and from 3 to about 18 ring atoms. Preferably heteroaromatic and heteroalicyclic groups contain from 5 to about 10 ring atoms. Suitable heteroaromatic groups in the compounds of the present invention contain one, two or three heteroatoms selected from N, O or S atoms and include, e.g., coumarinyl including 8-coumarinyl, quinolyl including 8-quinolyl, isoquinolyl, pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothienyl, benzothiazolyl, benzoxazolyl, quinazolinyl, quinoxalinyl, naphthyridinyl, and furopyridinyl. Suitable heteroalicyclic groups in the compounds of the present invention contain one, two or three heteroatoms selected from N, O or S atoms and include, e.g., pyrrolidinyl, tetrahydrofuryl, dihydrofuryl, tetrahydrothienyl, tetrahydrothiopyranyl, piperidyl, morpholinyl, thiomorpholinyl, thioxanyl, piperazinyl, azetidinyl, oxetanyl, thietanyl, homopiperidinyl, oxepanyl, thiepanyl, azepinyl, oxazepinyl, diazepinyl, thiazepinyl, 1,2,3,6-tetrahydropyridyl, 2-pyrrolinyl, 3- pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3H-indolyl, and quinolizinyl.

Preferably, the above mentioned groups that may be substituted are substituted at one or more available positions by one or more groups selected from R^{e}, -OR^{e}, =O, -NHR^{e}, -N(R^{e})₂, -C(=O)R^{e}, -C(=O)OR^{e}, -C(=O)NHR^{e},-C(=O)N(R^{e})₂, halogen, wherein each R^{e} is independently selected from hydrogen, and substituted or unsubstituted alkyl, alkenyl or alkynyl group.

However, in a preferred embodiment, the above mentioned groups that may be substituted are not substituted.

The term "salt" must be understood as any form of a compound of formula (I) according to the present invention in which said compound is in ionic form, or is in ionic form and coupled to a counter-ion (a cation or anion). Preferably, the salt is a pharmaceutically acceptable salt, i.e. a salt that is tolerated physiologically (preferably meaning that it is not toxic, particularly, as a result of the counter-ion) when used in an appropriate manner (i.e. in reasonable medical doses) for a treatment according to the present invention.

The term "stereoisomer" must be understood as an enantiomer, diastereomer, or a mixture thereof, such as a racemate, of a compound of formula (I) according to the present invention. Likewise, the term also encompasses geometric isomers about any double bonds present in the compound of formula (I), i.e. (E)-isomers and (Z)-isomers (trans and cis isomers). If the compound of formula (I) contains several double bonds, each double bond will have its own stereoisomerism, that could be the same as, or different to, the stereoisomerism of the other double bonds of the compound of formula (I). Furthermore, compounds of formula (I) may exist as atropisomers. All the stereoisomers including enantiomers, diastereoisomers, geometric isomers and atropisomers of the compounds of formula (I), and mixtures thereof, are considered within the scope of the present invention.

Furthermore, any compound of formula (I) according to the present invention may exist as tautomers. Specifically, the term tautomer refers to one of two or more structural isomers of a compound of formula (I) that exist in equilibrium and are readily converted from one isomeric form to the other. Common tautomeric pairs are amine-imine, amide-imidic acid such as lactam-lactim, or keto-enol.

The term "solvate" in accordance with this invention should be understood as meaning any compound of formula (I) according to the present invention in which said compound is bonded by a non-covalent bond to another molecule (normally a polar solvent), including especially hydrates and alcoholates, like for example, methanolate. A preferred solvate is the hydrate. Preferably, the solvate is a pharmaceutically acceptable solvate, i.e. a solvate that is tolerated physiologically (preferably meaning that it is not toxic, particularly, as a result of the solvating molecule) when used in an appropriate manner (i.e. in reasonable medical doses) for a treatment according to the present invention.

In a preferred embodiment, R¹ is selected from the group consisting of-C(=O)OR^{a}, -C(=O)NHR^{a}, -C(=O)N(R^{a})₂, -C(=O)R^{a}. More preferably, R¹ is-C(=O)OR^{a}. Most preferably, R¹ is -C(=O)OH.

In a preferred embodiment, R^{a} is H.

In a preferred embodiment, R² is selected from -OR^{c}, -NHR^{c} and -N(R^{c})₂. Preferably, R² is -OR^{c}. More preferably, R² is -OH.

In a preferred embodiment, R^{c} is H.

In a preferred embodiment, R³ is selected from substituted or unsubstituted alkyl, alkenyl and alkynyl group. Preferably, R³ is selected from substituted or unsubstituted C₁₋₆ alkyl, C₂₋₆ alkenyl and C₂₋₆ alkynyl, in particular unsubstituted C₁₋₆ alkyl, C₂₋₆ alkenyl and C₂₋₆ alkynyl. More preferably, R³ is selected from substituted or unsubstituted propyl, propenyl and propynyl, in particular unsubstituted propyl, propenyl and propynyl. More particularly, propenyl and propynyl are 1-propenyl and 1-propynyl.

More preferred is that R³ is substituted or unsubstituted C₂₋₆ alkenyl, in particular unsubstituted C₂₋₆ alkenyl, and even more preferably that R³ is substituted or unsubstituted propenyl, in particular unsubstituted propenyl. More particularly, propenyl is 1-propenyl.

In a preferred embodiment, R⁴ is selected from substituted or unsubstituted alkyl, alkenyl and alkynyl group. Preferably, R⁴ is selected from substituted or unsubstituted C₁₋₆ alkyl, C₂₋₆ alkenyl and C₂₋₆ alkynyl, in particular unsubstituted C₁₋₆ alkyl, C₂₋₆ alkenyl and C₂₋₆ alkynyl. More preferably, R⁴ is selected from substituted or unsubstituted propyl, propenyl and propynyl, in particular unsubstituted propyl, propenyl and propynyl. More particularly, propenyl and propynyl are 1-propenyl and 1-propynyl.

More preferred is that R⁴ is substituted or unsubstituted C₂₋₆ alkenyl, in particular unsubstituted C₂₋₆ alkenyl, and even more preferably that R⁴ is substituted or unsubstituted propenyl, in particular unsubstituted propenyl. More particularly, propenyl is 1-propenyl.

In a preferred embodiment, R⁵ is selected from substituted or unsubstituted alkyl, alkenyl and alkynyl group. Preferably, R⁵ is selected from substituted or unsubstituted C₃₋₉ alkyl, C₃₋₉ alkenyl and C₃₋₉ alkynyl, in particular unsubstituted C₃₋₉ alkyl, C₃₋₉ alkenyl and C₃₋₉ alkynyl. More preferably, R⁵ is selected from substituted or unsubstituted hexyl, hexenyl or hexynyl, in particular unsubstituted hexyl, hexenyl or hexynyl.

More preferred is that R⁵ is substituted or unsubstituted C₃₋₉ alkyl, in particular unsubstituted C₃₋₉ alkyl, and even more preferably that R⁵ is substituted or unsubstituted hexyl, in particular unsubstituted hexyl.

In a preferred embodiment, X is halogen. More preferably, X is Cl.

In an embodiment, the above preferences for R¹ and R² are combined. In another embodiment, the above preferences for R¹ and R³ are combined. In another embodiment, the above preferences for R¹ and R⁴ are combined. In another embodiment, the above preferences for R¹ and R⁵ are combined. In another embodiment, the above preferences for R¹ and X are combined. In another embodiment, the above preferences for R² and R³ are combined. In another embodiment, the above preferences for R² and R⁴ are combined. In another embodiment, the above preferences for R² and R⁵ are combined. In another embodiment, the above preferences for R² and X are combined. In another embodiment, the above preferences for R³ and R⁴ are combined. In another embodiment, the above preferences for R³ and R⁵ are combined. In another embodiment, the above preferences for R³ and X are combined. In another embodiment, the above preferences for R⁴ and R⁵ are combined. In another embodiment, the above preferences for R⁴ and X are combined. In another embodiment, the above preferences for R⁵ and X are combined.

In a particular embodiment, the compound of formula (I) is Compound 1 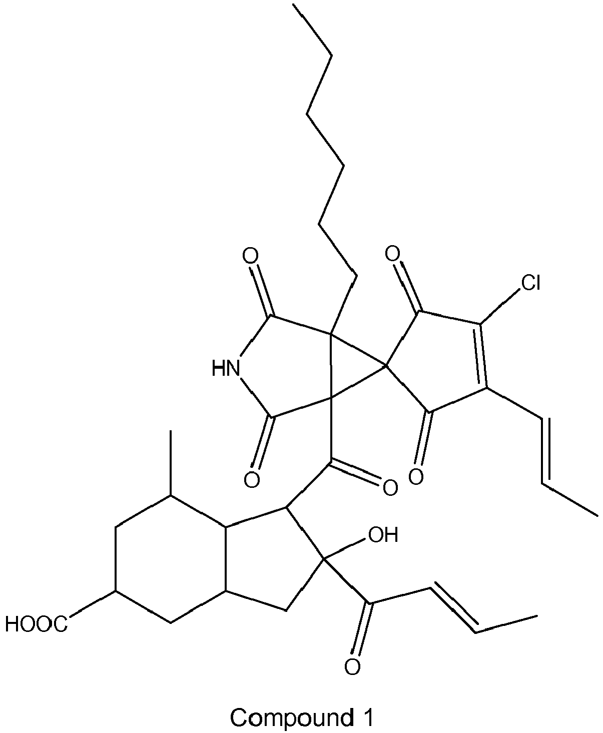 or a pharmaceutically acceptable salt, stereoisomer, or solvate thereof. This compound is numbered as follows:

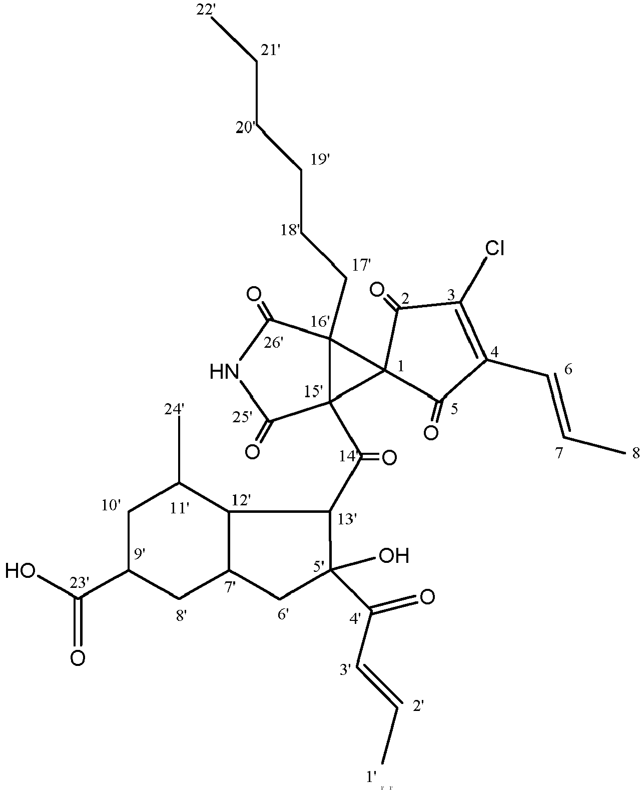

More particularly, the compound of formula (I) is the following stereoisomer of Compound 1 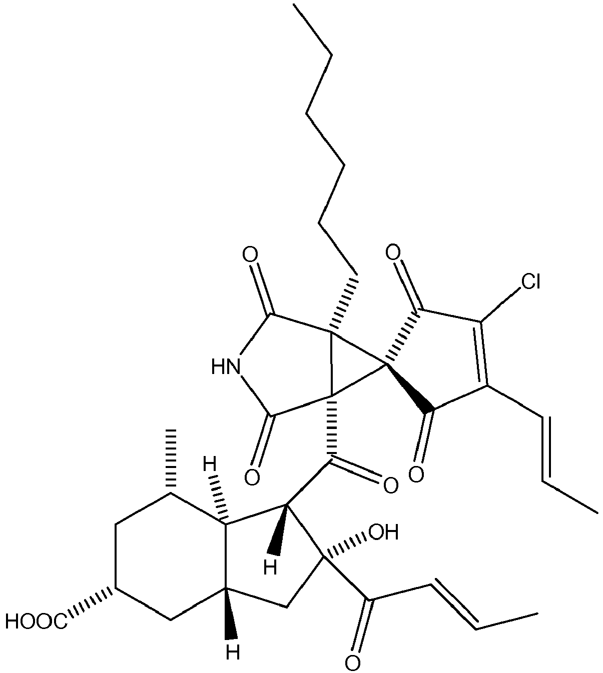 or a pharmaceutically acceptable salt or solvate thereof.

In another embodiment, the compound of formula (I) is not Compound 1.

The compounds of formula (I) of the present invention can be isolated from the fermentation broth of a microorganism, a fungal strain *Paraconiothyrium sp.* HL-78-gCHSP3-B005, a culture of which has been deposited in the Colección Española de Cultivos Tipo at the Universidad de Valencia, Spain under accession number CECT 20841. This deposit has been made under the provisions of the Budapest Treaty. The microorganism was isolated from a marine chordata sample collected in Guatemala. *Paraconiothyrium sp.* HL-78-gCHSP3-B005 cultured under controlled conditions in a suitable medium produces Compound 1. This strain is preferably grown in an aqueous nutrient medium, under aerobic and mesophilic conditions.

The compounds of formula (I) of the present invention, and more particularly Compound 1, can also be isolated from *H. velutinum* yone96, as described in Tanaka et al., J Org Chem, 2017, 82, 5574-5582.

While the above mentioned strains are preferred for the obtaining of Compound 1, the present invention is not restricted or limited to any particular strain or organism.

The compounds of formula (I) isolated from *Paraconiothyrium sp.* HL-78-gCHSP3-B005 or H. *velutinum* yone96 can be synthetically modified to arrive at further compounds of formula (I). Thus, hydroxyl groups can be acylated by standard coupling or acylation procedures, for instance by using acetic acid, acetyl chloride or acetic anhydride in pyridine or the like. Formate groups can be obtained by heating hydroxyl precursors in formic acid. Hydroxyl groups can be converted into halogen groups through intermediate sulfonates for iodide, bromide or chloride, or directly using a sulfur trifluoride for fluorides; or they can be reduced to hydrogen by reduction of intermediate sulfonates. Hydroxyl groups can also be converted into alkoxy groups by alkylation using an alkyl bromide, iodide or sulfonate, or into amino lower alkoxy groups by using, for instance, a protected 2-bromoethylamine. Ester groups can be hydrolyzed to carboxylic acids or reduced to aldehyde or to alcohol. Reaction of a carboxylic acid with thionyl chloride gives the corresponding acid chloride, which can be converted in an amide by reaction with the corresponding amine. Reaction of the acid chloride with an alcohol in presence of pyridine affords the corresponding ester, which can be easily converted in the corresponding alcohol by different reduction methods. Swern oxidation of the alcohol affords the corresponding aldehyde. Carboxylic acids can also be coupled with amines to provide amides by standard coupling or acylation procedures. Amido groups can be alkylated or acylated by standard alkylation or acylation procedures, for instance by using, respectively, KH and methyl iodide or acetyl chloride in pyridine or the like. Double bonds may be reduced such as by hydrogenation, epoxidated, or subjected to addition reactions. The procedures and reagents needed to prepare these derivatives are known to the skilled person and can be found in general textbooks such as March's Advanced Organic Chemistry 6th Edition 2007, Wiley Interscience.

It is also an object of the invention to use medicaments or pharmaceutical compositions comprising at least one compound of formula (I) as defined above, or a pharmaceutically acceptable salt, stereoisomer, or solvate thereof, and at least one pharmaceutically acceptable excipient, to treat NF-κB-related diseases or disorders.

The term "excipient" refers to components of a drug compound other than the active ingredient (definition obtained from the European Medicines Agency - EMA). They preferably include a "carrier, adjuvant and/or vehicle". Carriers are forms to which substances are incorporated to improve the delivery and the effectiveness of drugs. Drug carriers are used in drug-delivery systems such as controlled-release technology to prolong in vivo drug actions, decrease drug metabolism, or reduce drug toxicity. Carriers are also used to increase the effectiveness of drug delivery to the target sites of pharmacological action. Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker© 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

The pharmaceutical composition according to the present invention can be in any suitable form for its application in human beings and/or animals, preferably human beings, including infants, children and adults, and can be produced by means of conventional methods known by those skilled in the art, for example those described or mentioned in the Spanish and US Pharmacopoeias and similar reference texts. Common dosage form examples are solid dosage forms (tablets, pills, capsules, etc.) or liquid dosage forms (solutions, suspensions or emulsions).

The compound of formula (I) in the pharmaceutical composition may have different physical characteristics than its naturally occurring version. In particular, the presence of excipients can make the compounds have markedly different characteristics from the naturally occurring components. For instance, antioxidants allow improving the stability so that the compounds spoil much more slowly than the naturally occurring counterpart. For example, in the case of pharmaceutical compositions suitable for topical administration such as oils, creams or lotions, the compound may adhere to the skin much longer than its natural counterpart.

Administration of the compounds of the present invention can be intraperitoneal, intramuscular, intra-articular, intravenous, intra-arterial, intravesical, intraosseous, intracavernous, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, administration by means of needles or catheters with or without pump devices, topical administration, particularly dermal, transdermal or subcutaneous, or other routes for the application thereof.

In an embodiment, the administration is oral, intravenous, or intraperitoneal. In a more particular embodiment, the administration is intraperitoneal.

In an embodiment, the compound of formula (I) of the present invention is administered in water optionally together with ingredients that increase the solubility of the compounds, for example organic solvents such as dimethylsulfoxide, propylene glycol, polyethylene glycol, ethanol, glycerol, polyethylene glycol ricinoleate (Cremophor) or polysorbates, preferably Cremophor and/or ethanol.

In the context of the present invention, use of the compound formula (I) of the present invention is understood to be present or administered in therapeutically effective amounts. The physician will determine the most suitable dosage of the compounds and this will vary with the dosage form and the particular compound that is chosen, and it will furthermore vary with the patient undergoing treatment, e.g. with the age or medical history of the patient, or with the type of disease or condition that it being treated. When the composition is administered orally, larger amounts of the active agent will be required to produce the same effect as a smaller amount that is administered parenterally.

Similarly, the dosage regime will also depend on all the above mentioned factors. In an embodiment, the compound formula (I) of the present invention is administered daily, such as once a day, for example for four weeks or for at least four weeks, usually until the point in time when a physician notes appropriate disease control.

In an embodiment, the compound formula (I) is administered in one dose or in total daily doses in the range of between 0.001 µg/kg and 30 mg/kg, preferably between 0.001 mg/kg and 10 mg/kg, more preferably between 0.01 mg/kg and 10 mg/kg, preferably between 0.1 mg/kg and 10 mg/kg, preferably between 1 mg/kg and 5 mg/kg, preferably between 2 mg/kg and 4 mg/kg, and especially preferably between at about 3 mg/kg. Intraperitoneal administration is particularly preferred at these doses.

In an embodiment, the compound of formula (I) is administered at a concentration of between 0.01 and 10 mg, preferably of between 0.1 and 10 mg, more preferably of between 0.1 and 1 mg, in particular at about 0,6 mg, in each case per mL of solution of the compound of formula (I). Intraperitoneal administration is particularly preferred at these concentrations.

The above doses and concentrations are those administered to female C57BL/6 mice, such as those of about 17.8 g weight. The present invention covers said doses and concentrations as well as the equivalent doses and concentrations in humans, even if the claims are limited with the above dose or concentration values. The skilled person knows how to convert mouse doses into human doses, for instance following the Human Equivalent Dose Calculations established in the FDA's "Guidance for Industry - Estimating the Maximum Safe Starting Dose in Initial Clinical Trials for Therapeutics in Adult Healthy Volunteers" of July 2005, which is herein incorporated by reference.

The present invention refers to a compound of formula (I), or a pharmaceutically acceptable salt, stereoisomer, or solvate thereof, or a pharmaceutical composition comprising any of these, for use in the treatment of an NF-κB-related disease or disorder which is not cancer. An NF-κB-related disease or disorder is one mediated by NF-κB dysfunction, preferably by NF-κB overactivation. Preferably, the NF-κB-related disease or disorder is one involving overactivation of the NF-κB non-canonical pathway. More particularly, the disease or disorder is an inflammatory or an autoimmune or alloimmune disease or disorder, more specifically an NF-κB-related inflammatory or autoimmune or alloimmune disease or disorder. Preferably, the disease or disorder is an inflammatory or an autoimmune disease or disorder, more specifically an NF-κB-related inflammatory or autoimmune disease or disorder.

More preferably, the NF-κB-related disease or disorder is a NIK-related disease or disorder. A NIK-related disease or disorder is one mediated by NIK dysfunction, preferably by NIK overactivation. More particularly, the disease or disorder is an inflammatory or an autoimmune or an alloimmune disease or disorder, more specifically a NIK-related inflammatory or autoimmune or alloimmune disease or disorder. Preferably, the disease or disorder is an inflammatory or an autoimmune disease or disorder, more specifically a NIK-related inflammatory or autoimmune disease or disorder.

More particularly, the NF-κB-related disease or disorder or NIK-related disease or disorder is selected from rheumatoid arthritis; multiple sclerosis; asthma; inflammatory bowel disease; colonic inflammation; chronic obstructive pulmonary disease; diabetes and obesity, in particular diabetes- or obesity-associated inflammation; transplantation rejection, in particular GVHD; liver injury and liver fibrosis; dermatitis; systemic lupus erythematosus; and psoriasis.

The above mentioned diseases and disorders are well known in the art of medicine and will in any case have the meaning attributed to them in the 10th revision of the International Statistical Classification of Diseases and Related Health Problems (ICD-10) World Health Organization classification. See for instance rheumatoid arthritis (code M05-06) or multiple sclerosis (code G35).

In a more preferred embodiment of the present invention, the NF-κB-related disease or disorder or NIK-related disease or disorder is selected from rheumatoid arthritis, multiple sclerosis, asthma, inflammatory bowel disease, transplantation rejection, systemic lupus erythematosus and type 2 diabetes.

In a particularly preferred embodiment, the NF-κB-related disease or disorder or NIK-related disease or disorder is rheumatoid arthritis or multiple sclerosis. In a preferred embodiment, it is rheumatoid arthritis. In another preferred embodiment, it is multiple sclerosis.

In an embodiment, the NF-κB-related disease or disorder or NIK-related disease or disorder is not cancer or fungal infection. In an embodiment, the NF-κB-related disease or disorder or NIK-related disease or disorder is not cancer, hemangioma, retinopathy, neovascular glaucoma, macular degeneration, diabetes, psoriasis, rubeosis, arthritis, endometriosis or atherosclerosis. More specifically, it is not cancer, psoriasis, rheumatoid arthritis or diabetes. In another embodiment, transplantation rejection is preferably Graft-versus-host-disease, and in another embodiment these do not include corneal graft rejection.

As used herein, the term "treatment" or derivations thereof include the eradication, removal, reversion, alleviation, modification, or control of the NF-κB-related disease or disorder. In it broadest sense, the term "treatment" also comprises the prevention of the NF-κB-related disease or disorder. The term "prevention" or derivations thereof refer to the avoiding or minimizing of the onset or recurrence of the NF-κB-related disease or disorder. In an embodiment, the term "treatment" does not comprise prevention.

The subject which is treated is a human or animal, preferably a human subject.

In a particular embodiment, the compounds of formula (I) and pharmaceutical compositions of this invention may be used together with other drugs to provide a combined therapy. The other drugs may form part of the same composition, or be provided as a separate composition for administration at the same time or at different time.

For instance, in the treatment of multiple sclerosis, non-limiting examples of the other drug that can be used to provide a combination therapy are Interferon beta 1a or 1b, Glatiramer, Mitoxantrone, Natalizumab, a glucocorticoid, Fingolimod, cladribin, Teriflunomid, Fampridin, a HMG-CoA reductase inhibitor or a cannabinoid. On another hand, in the treatment of rheumatoid arthritis, non-limiting examples of the other drug that can be used to provide a combination therapy are DMARDs such as hydroxychloroquinesulfate, leflunomide, methotrexate, tofacitinib; abatacept, adalimumab, anakinra, etanercept, rituximab, infliximab-dyyb, golimumab, certolizumab pegol, tocilizumab, sarilumab, tofacitinib, baricitinib; glucocorticoids such as betamethasone or prednisone; NSAIDs such as celecoxib, diclofenac, ibuprofen, acetaminophen, tramadol, oxycodone or hydrocodone.

Having described the present invention in general terms, it will be more easily understood by reference to the following examples which are presented as an illustration and are not intended to limit the present invention.

### EXAMPLES

### Example 1: Obtaining of Compound 1 from HL-78-gCHSP3-B005

### Microorganism characterization

Taxonomic studies of the strain HL-78-gCHSP3-B005 are summarized as follows:

### Culture characteristics

Colonies reach 7 cm diameter in ten days at 25°C on potato dextrose agar in a culture chamber that maintains a humidity of 42%.

### Colony characteristics

Colonies growing fast, white to light brown, felt texture, becoming dark brown in concentric areas of submerged hyphae.

### Microscopy

Brown pycnidia approx. 100 µm in size, grouped around several areas of the external part of the colony. Simple conidiogenic cells, producing brown, septate, cylindrical-round ended, 8 x 4 µm, conidia.

Taxonomical determination was confirmed after a sequencing analysis of ITS1-5.8S-ITS2 ribosomal DNA region. Sequence shows a similarity percentage of 95% with the sequence of *Paraconiothyrium variabile.* Conidia characteristics and low similarity value does not confirm species level, but allows to identify the isolate as *Paraconiothyrium sp.*

The optimal temperature for growth on solid media is 24-28 °C. The pH range for growth is between 5 to 7. Growth was best with glucose and starch. Other carbon sources such as flour, glycerol, and dextrose can also be used.

Based on the preceding characteristics the culture has been determined as *Paraconiothyrium sp. The* fungal strain *Paraconiothyrium sp.* HL-78-gCHSP3-B005, was deposited in the Colección Española de Cultivos Tipo at the Universidad de Valencia, Spain under accession number CECT 20841.

### Production stage

*Paraconiothyrium sp.* HL-78-gCHSP3-B005 when cultured under controlled conditions in a suitable medium produces Compound 1. This strain is grown in an aqueous nutrient medium, under aerobic and mesophilic conditions, preferably between 24 and 28 °C at a pH 5.0 to 7.0.

A description of the process is as follows:

### Stock culture

A pure culture of *Paraconiothyrium sp.* HL-78-gCHSP3-B005 was kept frozen at -70°C in 20% glycerol.

### Preparation of inoculum

A well grown agar culture was used to inoculate 40 ml of seed medium containing 2% oat meal, 2% malt extract, 0.01% KH₂PO₄, 0.005% MgSO₄, and tap water in 250 ml shake flasks and cultured at 24 °C on a rotary shaker at 200 rpm. The flasks were incubated 48 hours in the dark, and used as a first stage inoculum.

### Fermentation

250 ml of a fermentation medium containing 2% sugar cane, 0.1% agar, 0.3% olive oil, 0.1% glycine, 0.3% KNO₃, 0.9% marine salts mixture and tap water in 2L Erlenmeyer flasks were inoculated with 10% of the first stage inoculum. The fermentation was carried for 7 days at 24 °C on a rotary shaker at 200 rpm in the dark.

Production of this compound can be monitored by HPLC or any other method with enough sensitivity.

### Isolation

Fermentation broth (4.5 L) of fungus HL-78-gCHSP3-B005 was filtered through Celite and the mycelial cake extracted twice with 2L of a mixture of EtOAc/MeOH (3:1). The resultant suspension was filtered and partitioned between EtOAc and water. The organic layer was taken to dryness and the crude extract (9.31 g) was fractionated by VFC (vacuum flash chromatography) on silica gel, eluted with a stepwise gradient of hexane/EtOAc/MeOH. Fractions containing Compound 1 (eluted with EtOAc/MeOH 9:1, 225 mg) were applied to a silica gel column and flash-chromatographed by elution with a CHCl₃/MeOH gradient. Fractions containing Compound 1 (eluted with CHCl₃/MeOH 93:7, 83 mg) were finally purified by semipreparative reversed-phase HPLC, affording 23 mg of pure Compound 1.

HPLC analysis is performed at room temperature using an analytical Symmetry C18 column (5 µm, 3.9x150mm) and as a mobile phase gradient from 50% MeOH/H₂O (1% formic acid) to 100% MeOH in 20 min and 100% MeOH 10 minutes more, a flow rate of 0.7 mL/min. and plotted at 220 nm, in this conditions the retention time for Compound 1 is 17.90 min.

### Example 2: Characterization of Compound 1

Compound 1 has a molecular formula of C₃₄H₄₀ClNO₉ established by APCI and API-ES mass spectra (pseudomolecular ion at *m*/*z* of 642 [M+H]+ and an isotopic peak at *m*/*z* of 644 with a ratio of 3:1), ¹³C NMR, and DEPT data. The complete assignments of ¹H and ¹³C NMR spectra of compound 1, were finally established by 2D NMR experiments (COSY, HSQC and HMBC). Its spectroscopic data are shown in table 1:

**Table 1. ¹H and ¹³C NMR Spectral Data of Compound 1 [δ (ppm), J_{HH} (Hz); CD₃OD]**

| Position | ¹³C (d) | ¹H (d) | HMBC |
|---|---|---|---|
| 1 | 48.0^{a} | | |
| 2 | 186.4 | | |
| 3 | 145.4 | | |
| 4 | 148.2 | | |
| 5 | 191.1 | | |
| 6 | 119.0 | 6.52 (1H, dq, 16.0, 1.7 Hz) | C3, C4, C5, C8 |
| 7 | 147.2 | 7.48 (1H, dq, 16.0, 7.0 Hz) | C4, C8 |
| 8 | 19.6 | 2.02 (3H, dd, 7.0, 1.7 Hz) | C6, C7 |
| 1' | 17.6 | 1.90 (3H, dd, 6.8, 1.6Hz) | C2', C3', C4' |
| 2' | 145.7 | 6.92 (1H, dq, 15.4, 6.8 Hz) | C1', C4' |
| 3' | 125.0 | 6.68 (1H, dq, 15.4, 1.6 Hz ) | C1', C4' |
| 4' | 201.6 | | |
| 5' | 86.2 | | |
| 6' | 45.4 | 1.46 (1H, m) | C4', C5', C7' |
| | | 2.05 (1H, m) | C4', C7', C12', C13' |
| 7' | 41.8 | 1.42 (1H, m) | C6' |
| 8' | 33.4 | 1.20 (1H, m) | C7', C12', C23' |
| | | 2.06 (1H, m) | C7', C12' |
| 9' | 43.3 | 2.40 (1H, m) | C23' |
| 10' | 38.3 | 1.22 (1H, m) | C8', C9', C11', C23', C24' |
| | | 1.93 (1H, m) | |
| 11' | 36.8 | 1.48 (1H, d, Hz) | |
| 12' | 51.4 | 1.83 (1H, d, 10.5 Hz) | C7', C8', C11', C13' |
| 13' | 61.2 | 4.10 (1H, d, 10.5 Hz) | C4', C5', C11', C12', C14' |
| 14' | 195.7 | | |
| 15' | 53.9^{a} | | |
| 16' | 49.7^{a} | | |
| 17' | 21.7 | 2.06 (1H, m) 2.27 (1H, ddd, 13.8, 11.7, 4.23 Hz) | C1, C15', C16', C18', C19', C26' |
| 18' | 27.3 | 1.05 (1H, m) | C19', C20' |
| | | 1.71 (1H, m) | C19', C20' |
| 19' | 29.1 | 1.28 (2H, m) | C20' |
| 20' | 31.2 | 1.26 (2H, m) | C18', C21', C22' |
| 21' | 22.3 | 1.28 (2H, m) | C20', C22' |
| 22' | 13.2 | 0.92 (3H, t, 7.1 Hz) | C20', C21' |
| 23' | 178.3 | | |
| 24' | 20.8 | 0.81 (3H, d, 6.6 Hz) | C10', C11', C12' |
| 25' | 168.6 | | |
| 26' | 171.9 | | |
| NH | | 10.4^{b} (1H, br s) | C15', C16', C25', C26' |
| 5'-OH | | 3.84b (1H, s) | C4', C5', C6', C13' |
| ^{a} interchangeable signals | | | |
| ^{b} Acetone-d6 spectra | | | |

### Example 3: Compound 1 biological activity

### NIK In Vitro Inhibitory Activity

To study NIK activity, transient transfection method of Jurkat cells and luciferase assays, as was previously described (Sánchez-Valdepeñas C et al., J.Immunol. 2006, 176(8), 4666-74), were used. Transcriptional activity was measured using reporter gene assays, which depend on NIK kinase activity, so the capacity of Compound 1 to inhibit the transcription activity of sequence CD28RE depending on NIK kinase activity was analysed.

Jurkat cells were transfected with CD28RE/AP-1 reporter gene with wildtype NIK-Flag and/or c-Rel or identical amount of empty DNA plasmid. After 4 h of incubation, RPMI medium containing 5% FBS cells were treated or not with different doses of the candidate compound and the incubation was continued for 16 h to complete transfection. Cells were then re-suspended in complete medium containing 5% FBS cells, harvested, lysed and luminescence measured for 10 seconds in a luminometer following the instructions in the "Dual-luciferase Assay System Kit" (Promega). Data are expressed in relative Firefly Luciferase Units (RLUs).

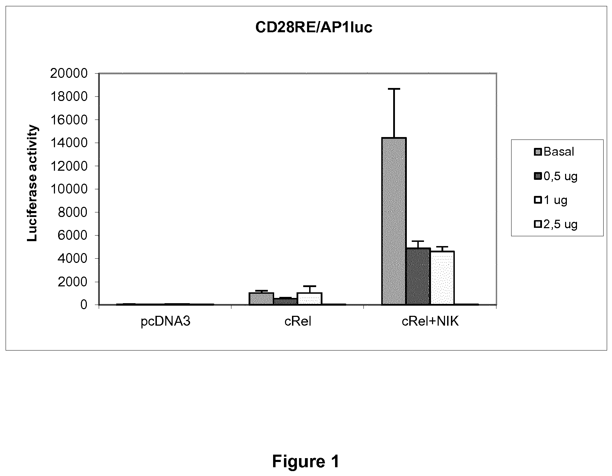

Figure 1 shows the results. It can be observed that basal NIK activity - and hence CD28RE/AP-1 mediated luminescence - was greatly reduced at all Compound 1 doses tested, and even abolished at relatively higher doses of the compound.

### In Vivo Activity in Animal Model

The aim of the study was to investigate the efficacy of a compound of formula (I) in an animal model of multiple sclerosis, i.e. Experimental Autoimmune Encephalomyelitis (EAE) in mouse.

### Study design

C57BI/6 female mice were injected subcutaneously (s.c.) at the flank on the back of the mouse with MOG35-55 emulsified in CFA (H37RA +IFA) on day 0. Pertussis toxin was injected on day 0 and 2 to boost the disease. Administration of Compound 1 was performed by intraperitoneal (i.p.) route daily from day 0. Positive control Cyclosporine A was administered by intraperitoneal (i.p.) route daily from day 0 until termination. Disease progression was followed macroscopically throughout the experiment. Blood was collected at termination, day 28 after disease induction. Serum was prepared from the blood samples and stored at - 80°C until shipment to Sponsor.

### Model description

Experimental Autoimmune Encephalomyelitis (EAE) in mice is a CD4+ T cell mediated autoimmune disease and the most widely accepted animal model of the human disease Multiple Sclerosis (MS). EAE is induced with one single injection of MOG35-55 emulsified with adjuvant and results in a highly reproducible onset about one week after induction. The most severe phase of disease is often observed between 15-20 days after induction and the experiment can be terminated 25-30 days after disease induction. Inflammatory lesions of the CNS causing peripheral paralysis are characteristic for EAE in mice. However, variations in disease characteristics are dependent on the specific mouse strain used and immunization protocol. Disease progression in the mice is followed by daily evaluation of disease symptoms using a well-recognised and evaluated scoring system.

### Materials and Methods

Reagents: Cremophor (Sigma, delivered by Sponsor); D-PBS (Life Technologies, 14190169); EtOH 70% (VWR, 83801.36, lot 171264020); EtOH (VWR, 83813.360, lot 17J254021); H37RA M. Tuberculosis (Difco, 231141, lot 7104681); Incomplete Freund's Adjuvant (IFA) (Difco, 263910, lot 7068986); Isoba vet 3.5% (Schering Plough Animal Health); MOG35-55 (produced by Red Glead Discovery, EXP-17-FG4314); Pertussis toxin from Bordetella pertussis (Calbiochem, MerckMillipore, 516560, lot 2972860); Sandimmun Neoral 100mg/ml (Novartis, 586107, lot H5203); Compound 1 was delivered as powder and stored at -20°C until day of administration; Vehicle (cremophor:EtOH:H₂O 10:10:80).

Equipment: Micro tubes (Eppendorf, VWR, 700-5239); Microvette 500 Z-gel (Sarstedt, 20.1344); Needles 0.6x25 mm (Terumo, Neolus, NN-2325R); Syringe 10ml Luer-Lok Tip (BD Biosciences, 300912); Syringe connection Connecta (BD Biosciences, 394600); Syringe 1ml (Codan, 62.1612). 2.3 Animals and Husbandry C57BI/6 mice (females, 6-8 weeks) were acquired from Janvier Europe.

Mice were housed in the animal facility at Medicon Village, Lund, Sweden, and kept at 12h light/dark cycles, in polystyrene cages (type III cages, 10 mice per cage) containing wood shavings and fed standard rodent chow and water ad libitum. The animals were acclimatized for approximately one week before initiation of experiment. Treatment groups were mixed within cages to avoid cage effects. The mice were identified by ear markings performed at day -1.

### Procedures

Determination of mean weight and group assignments: all mice were weighed one day prior to disease induction for determination of mean weight of the mice included in the experiment as well as for homogenous composition of the treatment groups. Mean weight day -1 also provided basis for determination of dosages for the cyclosporine treatment. Mean weight of all mice included in the experiment was determined to be 17.8 g on day -1.

Disease induction: EAE was induced day 0 with an emulsion containing 200 µg MOG₃₅₋₅₅ dissolved in PBS. The MOG protein solution was emulsified with Incomplete Freund's adjuvant (IFA) containing H37RA. The oil phase and the water phase were added to two separate syringes connected with a BD Connecta. The emulsion was pushed through the syringes until a white solid emulsion appeared. The emulsion was kept on ice during the preparation. Mice were anaesthetized with Isoba vet (3%) and oxygen, the root of the tail was cleaned with EtOH and the emulsion was injected subcutaneously (volume 100µl) at lower part of the back. A slight pressure was put on the injection site for 10 seconds after injection to prevent leakage of emulsion. Mice were removed from anaesthetics and health status including breathing was monitored until the mice were awake and until recovery from anaesthetics and injections was ensured. The animals were given booster injections day 0 and day 2, with 200ng Pertussis toxin intraperitoneally.

Dose preparation: Compound 1 was delivered as powder. At the day of administration 110µl of cremophor was added and the solution was mixed using a magnetic agitator with a small magnet. 110µl of absolute ethanol and the solution was again mixed using a magnetic agitator. Finally, 880µl water was added. The vehicle was prepared the same way. The solutions were injected i.p. in a total volume of approximately 100µl per animal. The volume was recalculated after every weight measurement to reach a dose at 3mg/kg for Compound 1. Positive control Cyclosporine A (Sandimmune) was purchased formulated at a concentration of 100 mg/ml and further diluted in PBS at a concentration of 0.89 mg/ml based on a mouse mean weight of 17.8g on day -1. The solution was injected i.p. in a total volume of 200 µl per animal.

Experimental groups and administration of test compound: Vehicle, Cyclosporine A and compound 1 were administered i.p. daily from day 0 to 10 mice. Compound 1 was administered at 3 mg/kg whereas Cyclosporine A was administered at a dose of 10 mg/kg. Table 1.

**Table 1. Administration volumes and concentrations.**

| Group | Treatment | Dose | Conc. Administered | Route admin. |
|---|---|---|---|---|
| 1 | Vehicle Control | n/a | n/a | i.p. |
| 2 | Cyclosporine A | 10 mg/kg | 0.89mg/ml | i.p |
| 3 | Compound 1 | 3 mg/kg | 0.6mg/ml | i.p |

Disease Evaluation: Disease was evaluated daily in a blinded fashion (scoring and administrations were performed by different people and scoring was performed only knowing the mouse number), starting day 5 until the end of the experiment according to the following criteria: 0 = healthy, 1 = tail weakness, 2 = tail paralysis, 3 = tail paralysis and mild waddle, 4 = tail paralysis and severe waddle, 5 = tail paralysis and paralysis of one limb, 6 = Tail paralysis and paralysis of a pair of limbs, 7 = tetraparesis or paralysis of three limbs and 8 = premorbid or dead.

Health evaluation: The general health of the mice was evaluated daily after disease induction. All mice were weighed 2 times per week, throughout the experiment as part of general health assessment.

Blood Samples: Blood was collected by cardiac puncture (under deep anesthesia) with a syringe and needle, at the end of the experiment on day 28. Blood was collected in gel containing micro tubes (Microvette 500 Z-Gel, Sarstedt 20.1344). The samples were centrifuged (10000xg, 5min) and serum was transferred to new prelabeled tubes. Serum samples were stored at -80oC until shipment to Sponsor.

Graphs and statistics: Graphs and statistical analysis were performed using Prism 5 for Mac OS X (GraphPad Software, San Diego, CA, USA). Results are presented as mean values ± SEM, if not otherwise stated. Statistics were calculated using a two-tailed non parametric Mann-Whitney test where p<0.05 was considered significant. * (or equivalent when letters are used in tables) represent a p-value< 0.05, ** (or equivalent when letters are used in tables) represent a p-value< 0.01 and *** (or equivalent when letters are used in tables) represent a p-value< 0.001 throughout the report.

### Results

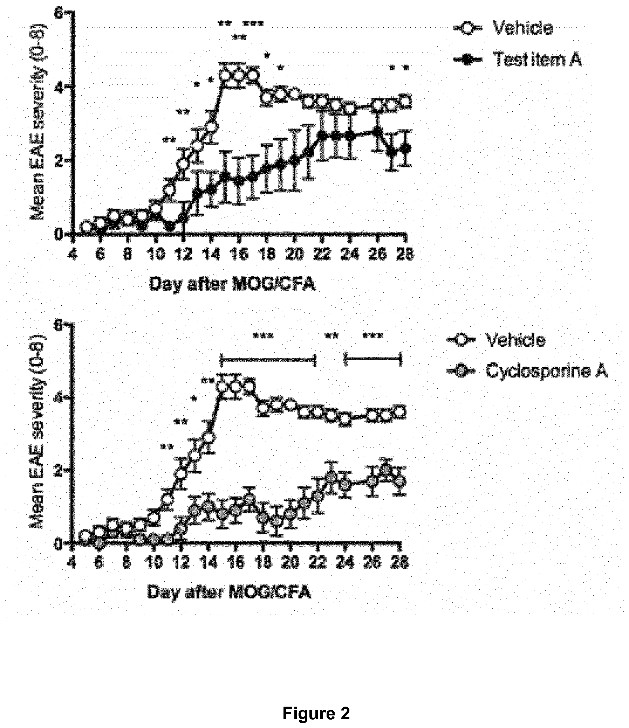

EAE severity as a function of time is shown at Figure 2. As can be clearly observed, the administration of Compound 1 (Test Item A) considerably reduced disease progression to a point comparable to that achieved by Cyclosporine A (it should be noted that Cyclosporine was administered at higher dose and concentration). Vehicle confirmed disease progression.

**Table 2. MaxES: Maximum EAE score; AUC: Area under curve.**

| Group | Treatment | MaxES | AUC |
|---|---|---|---|
| 1 | Vehicle Control | 4.8±0.3 | 61.5±2.5 |
| 2 | Cyclosporine A | 3.0±0.1 | 20.3±4.5 |
| 3 | Compound 1 | 3.3±0.6 | 34.1±8.3 |

## Claims

1. Compound of formula (I) 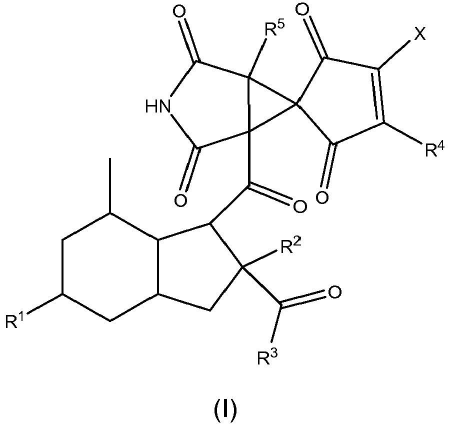 wherein
R¹ is selected from -C(=O)OR^{a}, -C(=O)NHR^{a}, -C(=O)N(R^{a})₂, -C(=O)R^{a}, and-CH₂OR^{a},
wherein each R^{a} is independently selected from hydrogen, and substituted or unsubstituted alkyl, alkenyl or alkynyl group;
R² is selected from R^{c}, -OR^{c}, -NHR^{c} and -N(R^{c})₂,
wherein each R^{c} is independently selected from hydrogen, and substituted or unsubstituted alkyl, alkenyl or alkynyl group;
R³, R⁴ and R⁵ are each independently selected from substituted or unsubstituted alkyl, alkenyl and alkynyl group;
X is H or halogen;
or a pharmaceutically acceptable salt, stereoisomer, or solvate thereof;
for use in the treatment of a NF-κB-related disease or disorder, with the proviso that the disease or disorder is not cancer.

2. The compound for use according to claim 1, wherein R¹ is -C(=O)OR^{a}, and R^{a} is as defined in claim 1.

3. The compound for use according to any preceding claim, wherein R² is-OR^{c}, and R^{c} is as defined in claim 1.

4. The compound for use according to any preceding claim, wherein R³ is selected from substituted or unsubstituted C₁₋₆ alkyl, C₂₋₆ alkenyl and C₂₋₆ alkynyl.

5. The compound for use according to any preceding claim, wherein R⁴ is selected from substituted or unsubstituted C₁₋₆ alkyl, C₂₋₆ alkenyl and C₂₋₆ alkynyl.

6. The compound for use according to any preceding claim, wherein R⁵ is selected from substituted or unsubstituted C₃₋₉ alkyl, C₃₋₉ alkenyl and C₃₋₉ alkynyl.

7. The compound for use according to any preceding claim, wherein X is halogen.

8. The compound for use according to any preceding claim, which is 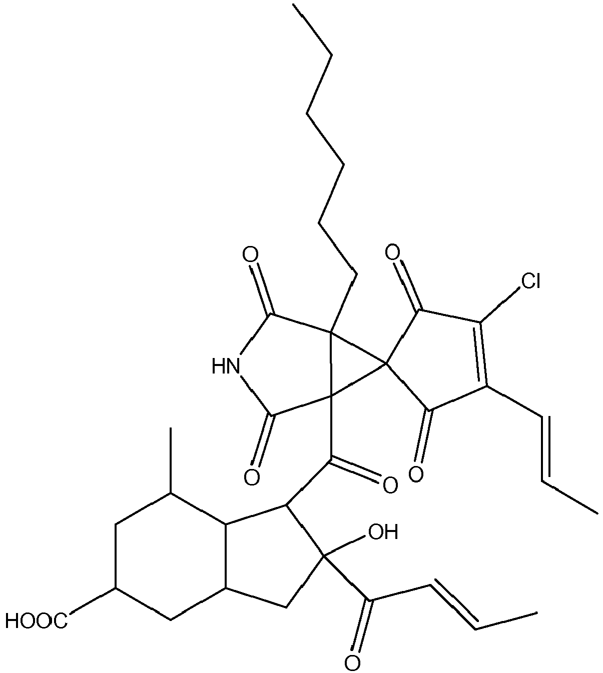 or a pharmaceutically acceptable salt, stereoisomer, or solvate thereof.

9. The compound for use according to any preceding claim, which is 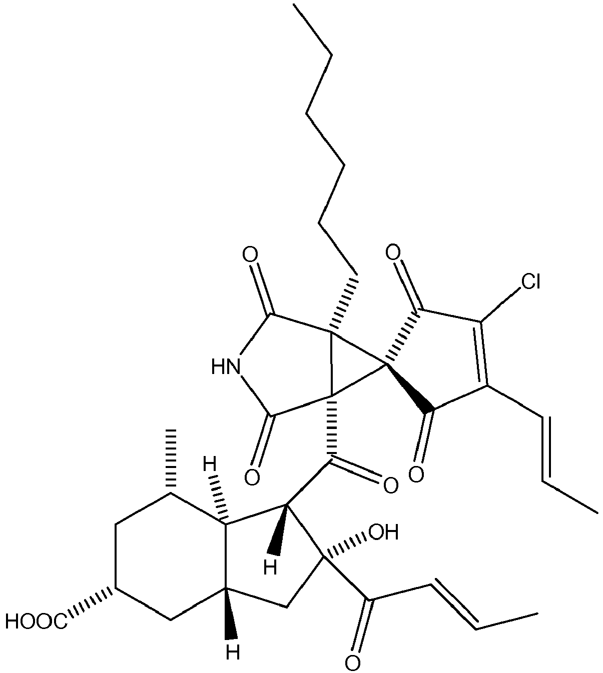 or a pharmaceutically acceptable salt or solvate thereof.

10. The compound for use according to any preceding claim, wherein the NF-κB-related disease or disorder is an inflammatory or autoimmune disease or disorder.

11. The compound for use according to any preceding claim, wherein the NF-κB-related disease or disorder is selected from rheumatoid arthritis; multiple sclerosis; asthma; inflammatory bowel disease; colonic inflammation; chronic obstructive pulmonary disease; diabetes and obesity, in particular diabetes- or obesity-associated inflammation; transplantation rejection, in particular GVHD; liver injury and liver fibrosis; dermatitis; systemic lupus erythematosus; and psoriasis.

12. The compound for use according to claim 11, wherein the NF-κB-related disease or disorder is selected from rheumatoid arthritis, multiple sclerosis, asthma, inflammatory bowel disease, transplantation rejection, psoriasis, dermatitis, systemic lupus erythematosus and type 2 diabetes.

13. The compound for use according to claim 11, wherein the NF-κB-related disease or disorder is rheumatoid arthritis or multiple sclerosis.

14. The compound for use according to claim 11, wherein the NF-κB-related disease or disorder is rheumatoid arthritis.

15. The compound for use according to claim 11, wherein the NF-κB-related disease or disorder is multiple sclerosis.
